# EUROPEAN PATENT SPECIFICATION

(11) **EP 2 992 933 B1**
(45) Date of publication and mention of the grant of the patent: **01.02.2017**
(21) Application number: 15183883.6
(22) Date of filing: 04.09.2015
(51) Int. Cl.: A61P 1/16, A61P 35/00, A61P 3/06, A61K 31/704, A61P 29/00, A23L 33/10, A61K 9/00

(54) **GINSENOSIDE F2 FOR PROPHYLAXIS AND TREATMENT OF LIVER DISEASE**
GINSENOSID F2 ZUR PROPHYLAXE UND BEHANDLUNG VON LEBERERKRANKUNGEN
GINSÉNOSIDE F2 POUR LA PROPHYLAXIE ET LE TRAITEMENT D'UNE MALADIE DU FOIE

(30) Priority: 05.09.2014 KR 20140118544
(43) Date of publication of application: 09.03.2016
(73) Proprietor: Korea Advanced Institute of Science and Technology, Daejeon 34141 (KR); Intelligent Synthetic Biology Center, Daejeon 34141 (KR)
(72) Inventor: JEONG, Won Il, 34121 Daejeon (KR); JUNG, Ju Yeon, 35397 Daejeon (KR); KIM, Sun Chang, 34141 Daejeon (KR); IM, Wan Taek, 34141 Daejeon (KR)
(74) Representative: Ström & Gulliksson AB

(56) References cited:
- NGUYEN HUU TUNG ET AL: "Pharmacological Effects of Ginseng on Liver Functions and Diseases: A Minireview", EVIDENCE-BASED COMPLEMENTARY AND ALTERNATIVE MEDICINE : ECAM, vol. 3, no. 6, 1 January 2012 (2012-01-01) , pages 410-7, XP055227880, United States ISSN: 1741-427X, DOI: 10.3748/wjg.v17.i18.2288

## Description

### BACKGROUND OF THE INVENTION

### 1. Field of the Invention

The present disclosure relates to a pharmaceutical composition, a health functional food, and a feed composition including ginsenoside F2 for prevention, improvement, or treatment of liver disease.

### 2. Description of the Related Art

Alcoholic liver disease may be largely classified into alcoholic fatty liver, alcoholic hepatitis, and alcoholic cirrhosis according to clinical symptoms, and a daily alcohol consumption exceeding 80 grams is known to increase the risk of liver disease, although there are individual variables of genetic characteristics and sex.

In particular, alcoholic fatty liver is caused by fat accumulation in the liver due to excessive production of fatty acid resulting from alcohol metabolism, and fatty liver is defined as an accumulation of lipid in the liver exceeding 5% of liver weight. Fat is mainly accumulated in the form of triglyceride.

Alcoholic fatty liver disease is known to be caused by many factors. First, excessive NADH generated during alcohol metabolism causes inhibition of fatty acid oxidation and increases fatty acid concentration in the liver, leading to accumulation of triglyceride. Further, a toxic intermediate, acetaldehyde produced in the alcohol metabolism binds with intracellular proteins to block secretion of proteins from the liver, and also induces lipid peroxidation and damages cell membrane to block secretion of lipoproteins from the liver, leading to accumulation of triglyceride in the liver. Fat accumulation in the liver by alcohol intake is recognized as a very important early sign in the development of liver diseases (hepatitis, liver cirrhosis, and liver cancer).

Known substances capable of inhibiting alcoholic fatty liver are substances having an antioxidant activity such as glutathione, 2-mecaptopropioylglycine, 3-amino-1,2,4-triazole, N,N'-diphenyl-p-phenylenediamine, etc., substances promoting synthesis of phospholipid and glutathione such as S-adenosyl-L-methionine, betaine, etc., and substances promoting alcohol metabolism such as L-glycine, L-cysteine, etc. (Korean Patent Publication No. 2012-0138392). However, there is still a demand for therapeutic agents for alcoholic liver disease having excellent efficacy without side effects.

On the other hand, it is known that ginsenoside F2 induces cell apoptosis accompanied by autophagy in breast cancer stem cells (CSCs) to exhibit anti-cancer effects on breast cancer (Mai TT et al. 2012; 28;321 (2):144-53), and ginsenoside F2 exhibits anti-cancer effects on glioblastoma in xenograft model in SD rats (Shin JY et al. 2012; 36(1):86-92). It is also known that ginsenoside F2 can be used as a cosmetic composition for improving skin wrinkles, skin whitening, or acne (Korean Patent Publication No. 2014-0013795). However, there have been no reports of therapeutic effects of ginsenoside F2 on liver diseases.

NGUYEN HUU TUNG ET AL: "Pharmacological Effects of Ginseng on Liver Functions and Diseases: A Minireview", EVIDENCE-BASED COMPLEMENTARY AND ALTERNATIVE MEDICINE: ECAM, vol. 3, no. 6, 1 January 2012, pages 410-417 discloses that ginsenoside Re (40 mg/kg) attenuates alcoholic fatty liver disease through regulation of AMPK and mitogen-activated protein kinase (MAPK) pathways in alcoholic-fed ICR mice.

With this background, the present inventors have made many efforts to develop a method of treating liver diseases. As a result, they have found that ginsenoside F2 extracted from ginseng protects alcohol-mediated liver injury, and also inhibits synthesis and accumulation of triglyceride and inflammation in the liver, and thus it can be used for the prevention or treatment of various alcoholic or non-alcoholic liver diseases, thereby completing the present invention.

### SUMMARY OF THE INVENTION

An object of the present invention is to provide a pharmaceutical composition for preventing or treating liver diseases, including ginsenoside F2.

Another object of the present invention is to provide a health functional food for preventing or improving alcoholic liver diseases, including ginsenoside F2.

Still another object of the present invention is to provide a feed composition for preventing or improving liver diseases, including ginsenoside F2.

Still another object of the present invention is to provide a method of treating liver diseases, including the step of administering the pharmaceutical composition to a subject.

### BRIEF DESCRIPTION OF THE DRAWINGS

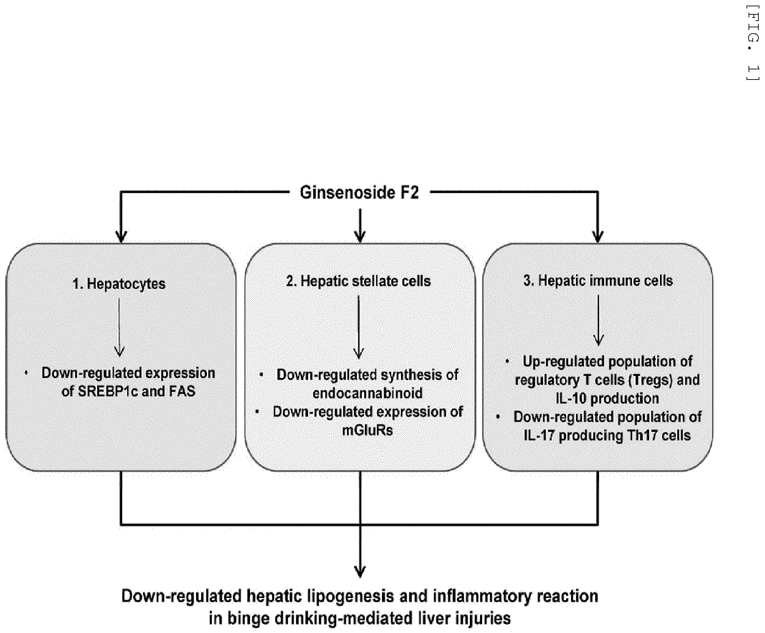

FIG. 1 shows a mechanism of ginsenoside F2 in the prevention or treatment of liver disease, in which data shown are presented as mean±SEM; *P < 0.05, **P < 0.01 versus the corresponding control (statistical analysis is the same as above);
FIG. 2 shows the results of a serological test (ALT (a), AST (b), TG (c), and total cholesterol levels (d)) according to ginsenoside F2 treatment;
FIGS. 3a to 3c show histological changes in the liver tissue according to ginsenoside F2 treatment;
FIGS. 4a to 4c show changes in the lipogenic index (SREBP1c, FAS, CB1R, DAGL-α,β, FAAH, pAMPK, etc.) of hepatocytes and hepatic stellate cells according to ginsenoside F2 treatment;
FIGS. 5a to 5g show lipogenesis-inhibiting effects of ginsenoside F2 and a mechanism thereof in hepatocytes and hepatic stellate cells;
FIGS. 6a to 6e show inhibitory effects of ginsenoside F2 on hepatic inflammatory changes by increasing regulatory T cells;
FIGS. 7a to 7c show inhibitory effects of ginsenoside F2 on differentiation of naive T cells into IL-17-producing Th17 cells; and
FIGS. 8a to 8h show loss of a liver protective effect of ginsenoside F2 in IL-10-deficient mice.

### DETAILED DESCRIPTION OF THE PREFERRED EMBODIMENTS

In order to achieve the above objects, an aspect of the present invention provides a pharmaceutical composition for preventing or treating liver diseases, including ginsenoside F2.

Ginsenoside F2 is a tetracyclic PPD (protopanaxadiol) saponin represented by the following Chemical Formula 1, and one of minor saponins showing higher absorption and efficacy than major saponins.

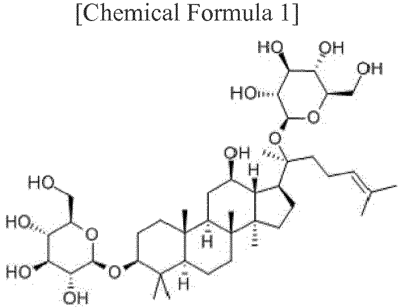

In the present invention, ginsenoside F2 may be purchased from a commercially available source, or isolated from ginseng cultured or collected in nature or converted from isolated ginsenoside. Alternatively, ginsenoside F2 synthesized by a synthetic method may be used. However, any ginsenoside F2 may be used without limitation, as long as it exhibits therapeutic or prophylactic effects on liver disease.

The liver disease may be, for example, selected from the group consisting of hepatitis, cirrhosis, fatty liver, hepatic insufficiency, and liver cancer, but is not limited thereto.

Hepatitis means inflammation of hepatocytes and hepatic tissues, and may progress to cirrhosis or liver cirrhosis when death and regeneration of hepatocytes due to chronic hepatitis are repeated for a long period of time to increase fibrous tissues and regenerative nodules in the liver. When cirrhosis reaches an advanced stage, it may cause complications such as hepatic encephalopathy, esophageal varix, etc.

Fatty liver means fat accumulation exceeding 5%, which is a normal liver fat content. The fatty liver may be alcoholic fatty liver, or non-alcoholic fatty liver caused by obesity, diabetes, or drugs.

Hepatic insufficiency means a condition characterized by decreased synthetic function and decreased detoxification, and is present together with hepatitis and cirrhosis.

The liver disease may be diagnosed by AST, ALT, ALP, GGT, or Bilirubin.

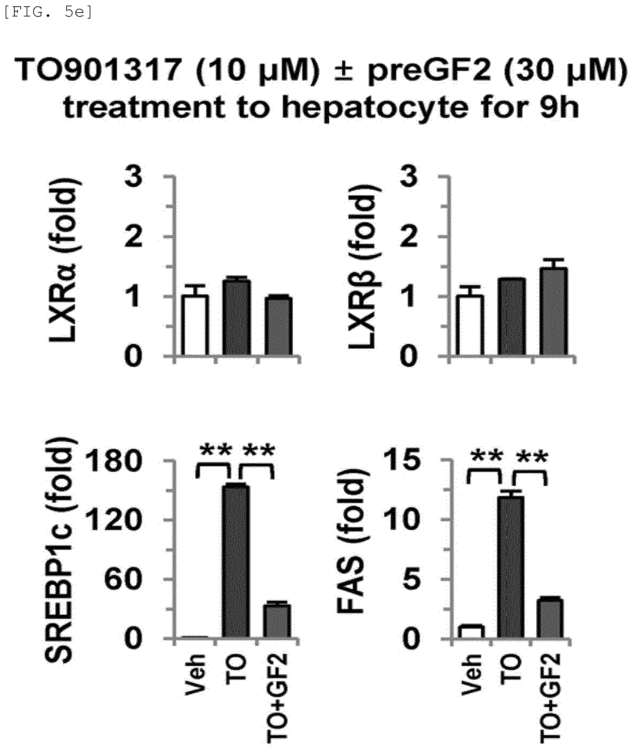

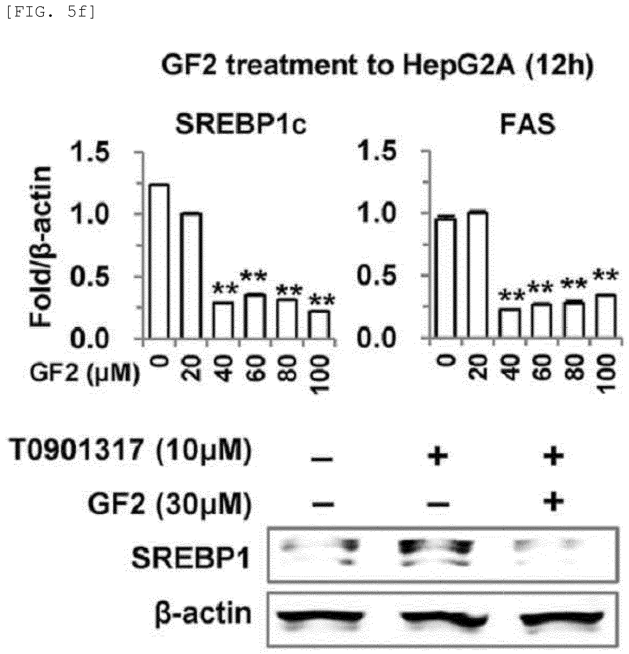

The present inventors confirmed that ginsenoside F2 inhibits expression of lipogenic genes SREBP1c and FAS increased by TO901317, which is an agonist of a lipogenesis-stimulating protein LXR (liver X receptor) (Example 2, FIGS. 5e, 5f to 5g). Therefore, it was confirmed that ginsenoside F2 exhibits excellent effects of inhibiting lipogenesis in hepatocytes and hepatic stellate cells, and in particular, ginsenoside F2 inhibits alcohol-induced endocannabinoid generation to reduce CB1R signal transduction in adjacent hepatocytes, leading to inhibition of lipogenesis in the liver. Accordingly, the pharmaceutical composition including ginsenoside F2 of the present invention may be used to effectively prevent or treat non-alcoholic and alcoholic liver diseases caused by liver fat accumulation.

The liver disease may also be alcoholic liver disease. The alcoholic liver disease means a liver disease caused by heavy drinking.

In alcoholic hepatitis, oxidative stress and various inflammatory mediators (TNF, proinflammatory cytokine, IL-1β, IL-6, or IL-8) are increased due to excessive alcohol consumption to generate reactive metabolites and oxygen species, which affect the defense mechanism of hepatocytes. Consequently, a normal liver regeneration mechanism is damaged to cause hepatotoxicity (McClain CJ et al., Semin Liver Dis 1999; 19:205-219).

Alcoholic fatty liver (AFLD) is caused by accumulation of triglyceride in the liver due to excessive alcohol consumption. Alcohol influences carbohydrate and lipid metabolism to increase the NADH/NAD⁺ ratio and precursors required for lipogenesis in hepatocytes, leading to increased triglyceride synthesis and decreased triglyceride degradation (Neuman MG et al., Exp Mol Pathol 95: 376-384), and excessively produced acetaldehyde impairs functions of major proteins and lipids of hepatocytes to induce a reduction in protein synthesis and accumulation of triglyceride, leading to injury of hepatocytes (Reuben A. 2008. Curr Opin Gastroenterol 24: 328-338). Long-term exposure to alcohol may develop fatty liver into hepatitis, cirrhosis, or liver cancer.

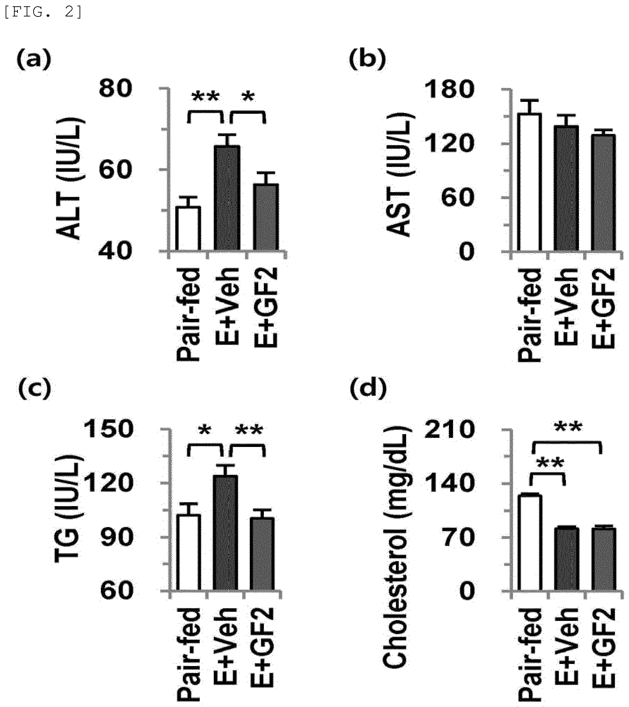

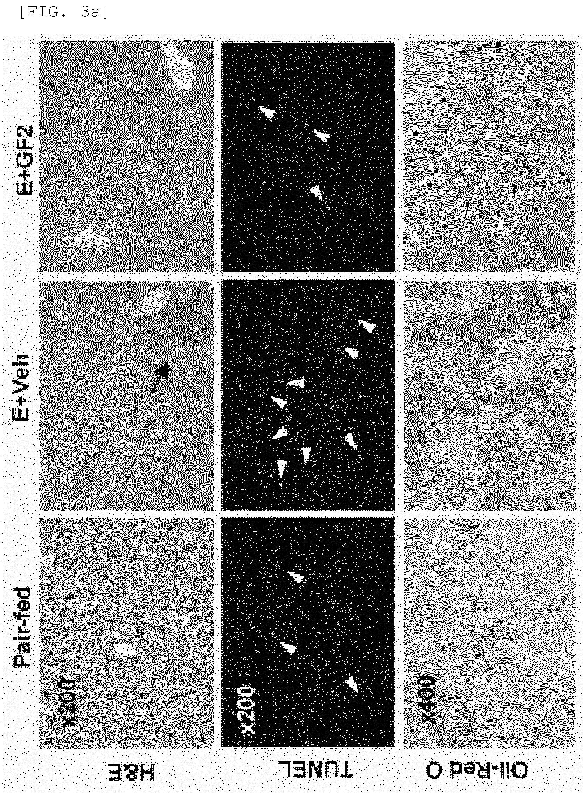

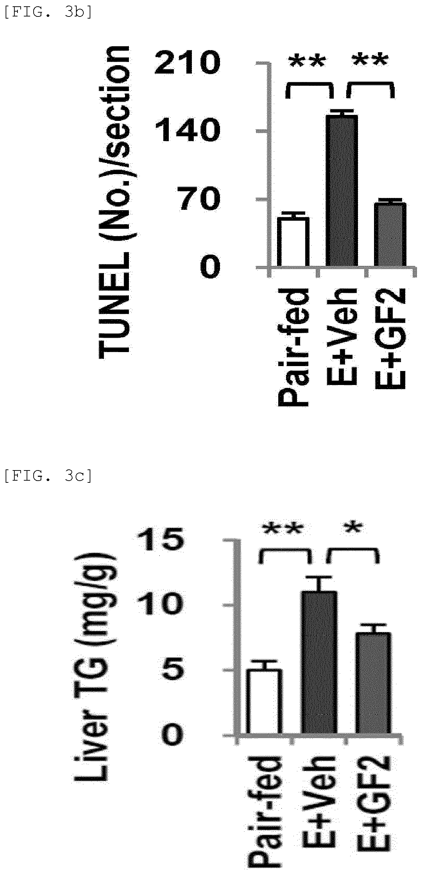

The present inventors performed a serological test to confirm that serum ALT and AST levels increased by alcohol are reduced by ginsenoside F2 (Example 1, FIG. 2), and they also performed liver tissue staining to confirm that inflammation and cell damage caused by ethanol administration are reduced by ginsenoside F2 (FIGS. 3a and 3b).

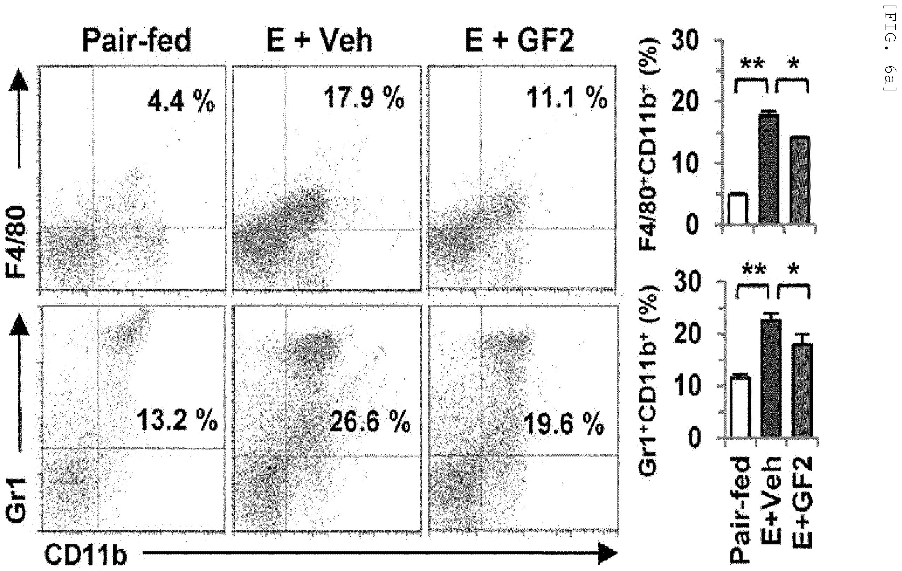

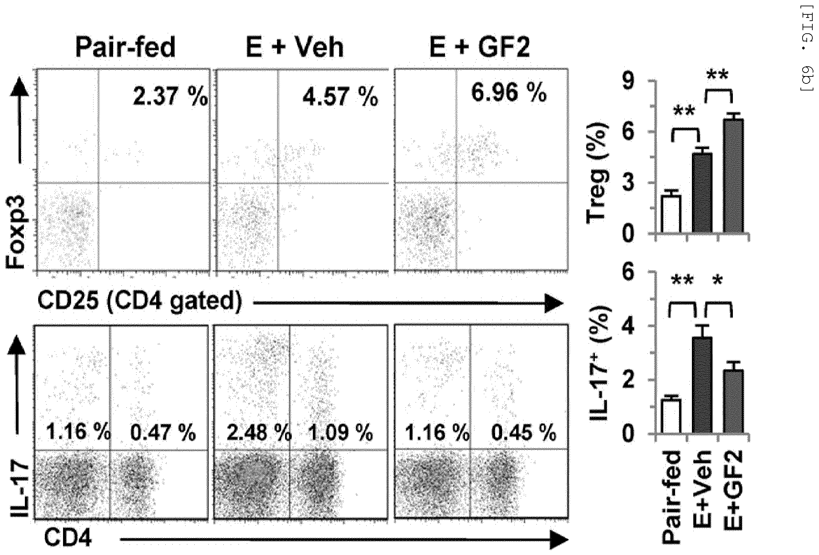

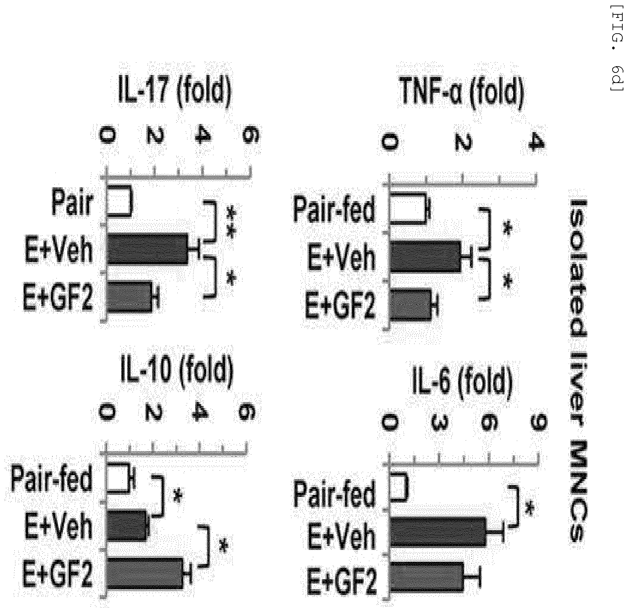

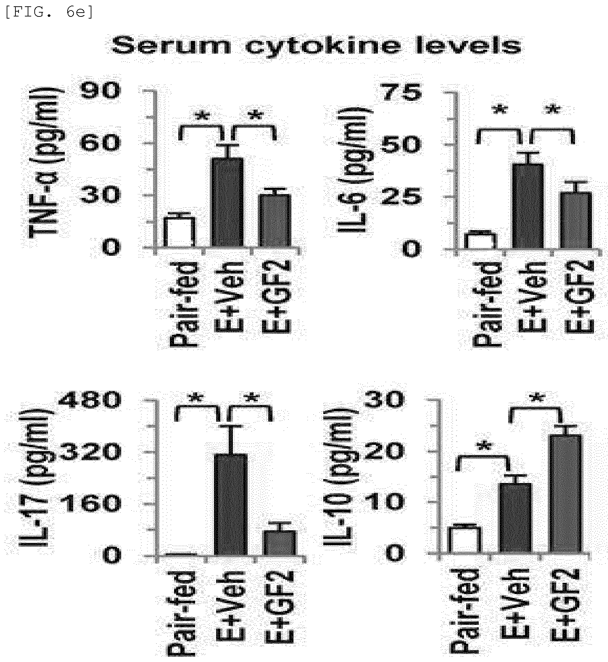

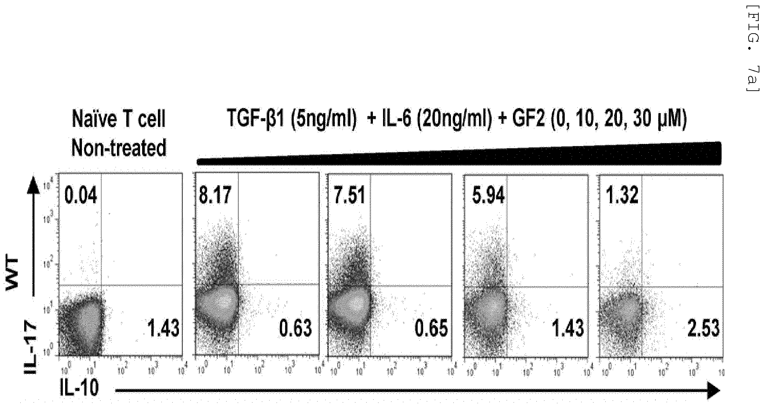

It was also confirmed that ginsenoside F2 increases distribution of regulatory T cells (Tregs) inhibiting activity of inflammatory cells such as macrophages or neutrophils in alcohol-mediated hepatitis, thereby protecting hepatitis (FIGS. 6a and 6b), and ginsenoside F2 inhibits expression of inflammatory cytokines TNF-α, IL-6, IL-17 increased by alcohol, and it increases expression of anti-inflammatory cytokine IL-10 (FIGS. 6d and 6e). Furthermore, ginsenoside F2 was confirmed to inhibit differentiation of naive T cells into proinflammatory Th17 cells expressing IL-17 (Example 4, FIGS. 7a to 7c).

Therefore, ginsenoside F2 may ameliorate liver inflammation caused by alcohol and liver injury.

Furthermore, the present inventors performed a serological test to confirm that serum triglyceride (TG) and total cholesterol levels increased by alcohol are reduced by ginsenoside F2 (Example 1, FIG. 2), and they also performed liver tissue staining to confirm that triglyceride accumulation in the liver increased by ethanol administration is reduced by ginsenoside F2 (FIGS. 3a and 3c).

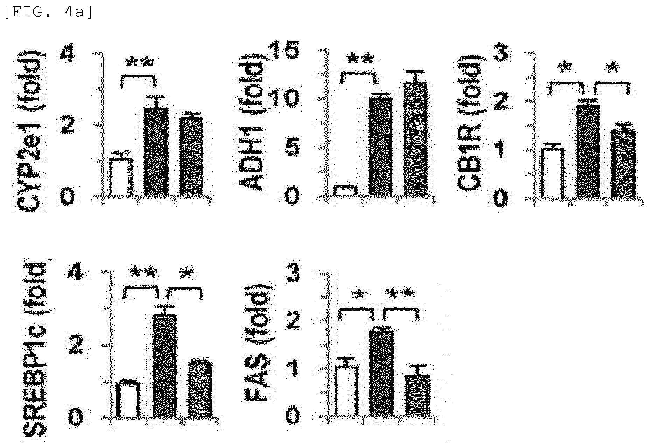

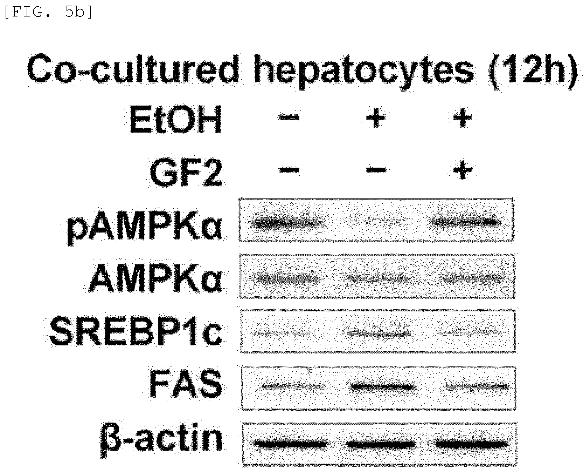

It was also confirmed that increased expression of SREBP1c and FAS lipogenic transcription factors in hepatocytes and hepatic stellate cells; CB1R, known to be critical for fatty acid synthesis in hepatocytes; and DAGL-α,β, stimulating endocannabinoid synthesis, is inhibited by ginsenoside F2, and decreased expression of fatty acid hydrolase FAAH and fatty acid oxidation-stimulating pAMPK is inhibited by ginsenoside F2 (FIGS. 4a to 4c, FIGS. 5b to 5d), suggesting that ginsenoside F2 may be used to effectively prevent or treat liver diseases by preventing or inhibiting fat synthesis and accumulation in the liver.

In an embodiment, therefore, the ginsenoside F2 may be ginsenoside F2 capable of inhibiting fat accumulation in the liver, and the ginsenoside F2 may be ginsenoside F2 capable of inhibiting hepatic inflammation by increasing distribution of regulatory T cells (Tregs). In another embodiment, the ginsenoside F2 may be ginsenoside F2 capable of increasing expression of anti-inflammatory cytokine IL-10. Further, the ginsenoside F2 may be ginsenoside F2 capable of inhibiting differentiation of naive T cells into Th17 cells. Furthermore, the ginsenoside F2 may be ginsenoside F2 capable of inhibiting LXR (liver X receptor) activity.

As used herein, the term "prevention" refers to all of the actions by which occurrence of liver disease is restrained or retarded by administration of ginsenoside F2 according to the present invention to a subject.

As used herein, the term "treatment" refers to all of the actions by which the symptoms of liver disease have improved or been modified favorably by administration of the composition of the present invention to a subject suspected of having liver disease.

The pharmaceutical composition of the present invention may be used as a single formulation or as a complex formulation prepared by further including a drug which is approved to have therapeutic effects on liver diseases, and the composition may be prepared as a unit dosage form or a multidose container by formulating it using a pharmaceutically acceptable carrier or excipient.

As used herein, the term "pharmaceutically acceptable carrier" refers to a carrier or a diluent that does not cause significant irritation to an organism and does not abrogate the biological activity and properties of the administered compound. A kind of carrier usable in the present invention is not particularly limited, and any carrier may be used as long as it is generally used in the art and is pharmaceutically acceptable. Non-limiting examples of the carrier may include normal saline, sterile water, Ringer's solution, buffered saline, an albumin injection solution, a dextrose solution, a maltodextrin solution, glycerol, ethanol, etc. One or a mixture of two or more thereof may be used. If necessary, another general additive such as an antioxidant, a buffer, and/or a bacteriostatic agent may be further added and used, and the composition may be formulated into injection formulations such as an aqueous solution, a suspension, an emulsion, or pills, capsules, granules, or tablets by additionally adding a diluent, a dispersant, a surfactant, a binder, a lubricant, etc., and then used.

Further, the pharmaceutical composition of the present invention may include a pharmaceutically effective amount of ginsenoside F2. As used herein, the term "pharmaceutically effective amount" refers to an amount sufficient to treat diseases, at a reasonable benefit/risk ratio applicable to any medical treatment. Generally, the pharmaceutical composition of the present invention may be administered in an amount of 0.001 mg/kg to 1000 mg/kg, preferably 0.05 mg/kg to 200 mg/kg, and more preferably 0.1 mg/kg to 100 mg/kg singly or divided into several times per day. However, with respect to the objects of the present invention, the specific therapeutically effective dose level for any particular patient may vary depending on a variety of factors such as the type and degree of the response to be achieved, the specific composition, including whether another agent, if any, is employed, the age, body weight, general health, sex and diet of the patient, the time of administration, route of administration, and rate of excretion of the composition, the duration of the treatment, drugs used in combination or coincidental with the specific composition, and similar factors well known in the medical arts.

The pharmaceutical composition of the present invention may be administered alone or in combination with other therapeutic agents, and it may be administered sequentially or simultaneously with conventional therapeutic agents. The composition may be administered in a single or multiple dosage form. It is important to administer the composition in the minimum amount that may exhibit the maximum effect without causing side effects, in view of all the above-described factors, and this amount can be easily determined by a person skilled in the art.

As used herein, the term "administration" refers to introduction of the pharmaceutical composition of the present invention into a patient using any suitable method. The composition of the present invention may be orally or parenterally administered via any of the common routes, as long as it is able to reach the desired tissue.

The administration mode of the pharmaceutical composition according to the present invention is not particularly limited, and the administration may be performed according to the mode generally used in the art. For non-limiting examples of the administration mode, the composition may be administered orally or parenterally. The pharmaceutical composition according to the present invention may be prepared into various formulations according to the desired administration mode.

With regard to the administration frequency, the composition of the present invention may be, but is not particularly limited to being, given once a day or several times a day as a divided dosage.

Still another aspect of the present invention provides a method of preventing or treating liver diseases, including the step of administering the composition to a subject suspected of having liver disease. For example, provided is a method of preventing or treating liver diseases, including the step of administering the composition to a subject suspected of having liver disease, excluding humans.

In this regard, the ginsenoside F2 and liver disease are the same as described above.

As used herein, the term "subject" may refer to all kinds of animals including humans, which already have liver disease or at risk for the disease. The animals may be mammals such as cattle, horses, sheep, pigs, goats, camels, antelope, dogs, and cats, which are in need of treatment for similar symptoms, as well as humans, but are not limited thereto.

Specifically, the prevention or treatment method of the present invention may include the step of administering a pharmaceutically acceptable amount of the composition into a subject having liver disease or at risk for the disease.

As used herein, the term "administration" refers to introduction of the pharmaceutical composition of the present invention into a patient using any suitable method. The composition of the present invention may be orally or parenterally administered via any of the common routes, as long as it is able to reach the desired tissue.

Still another aspect of the present invention provides a health functional food for preventing or improving liver disease, including ginsenoside F2.

The ginsenoside F2 and liver disease are the same as described above.

The ginsenoside F2 is a natural substance which may be extracted from ginseng, white ginseng, wild ginseng, etc. Since ginseng has been ingested for a long period of time, its safety has been approved. Therefore, it may be eaten raw or prepared in a food which is intended to be used for preventing or improving liver disease.

The term "health functional food" is the same term as food for special health use (FoSHU), and refers to a food having high medicinal and medical effects, which is processed to effectively exert a body-regulating function and to supply nutrients. The food may be prepared in various forms such as tablet, capsule, powder, granule, liquid, pill, etc., so as to provide a useful effect of preventing or improving liver disease.

The food composition of the present invention may further include a sitologically acceptable carrier.

A kind of the food to which the composition including ginsenoside F2 of the present invention may be added is not particularly limited, and examples thereof may include drinks, gums, teas, vitamin complexes, health supplement foods, etc. The food composition may further include other components which do not interfere with the effect of preventing or improving liver disease, and a kind thereof is not particularly limited. Like common foods, the food composition may include, for example, various herbal extracts, sitologically acceptable food auxiliary additives, or natural carbohydrates as additional components.

The food auxiliary additives may be added during the preparation of health functional foods in various formulations, and may be selected properly by those skilled in the art. Examples thereof may include a variety of nutrients, vitamins, minerals (electrolytes), synthetic and/or natural flavoring agents, colorants and fillers, pectic acid or salts thereof, alginic acid or salts thereof, organic acids, protective colloidal thickening agents, pH modifiers, stabilizers, preservatives, glycerin, alcohols, carbonating agents used in carbonated beverages, etc., but the kind is not limited to these examples.

In this regard, the content of the extract included in the food may be, is not particularly limited to, 0.01% to 100% by weight, and more preferably 1% to 80% by weight, based on the total weight of the food composition.

When the food is a drink, it may be included in an amount of 1 g to 30 g, preferably 3 g to 20 g, based on 100 mL. Also, the composition may further include an additive which is commonly used in food compositions to enhance flavor, taste, color, and the like. For example, the composition may include vitamins A, C, D, E, B1, B2, B6 and B12, niacin, biotin, folate, and pantothenic acid. The composition may also include a mineral, such as zinc (Zn), iron (Fe), calcium (Ca), chromium (Cr), magnesium (Mg), manganese (Mn), and copper (Cu). The composition may also include an amino acid, such as lysine, tryptophan, cysteine, and valine. The composition may also be supplemented with food additives, including antiseptics (e.g., potassium sorbate, sodium benzoate, salicylic acid, sodium dehydroacetate, etc.), disinfecting agents (e.g., bleaching powder and high-test bleaching powder, sodium hypochlorite, etc.), antioxidants (e.g., butylhydroxyanisole (BHA), butylhydroxytoluene (BHT), etc.), colorants (e.g., tar dye, etc.), color fixing agents (e.g., sodium nitrate, sodium nitrite, etc.), bleaching agents (e.g., sodium sulfite), seasoning agents (e.g., MSG, sodium glutamate, etc.), sweeteners (e.g., dulcin, cyclamate, sodium saccharine, etc.), flavoring agents (vanillin, lactones, etc.), blowing agents (alum, potassium D-bitartrate, etc.), fortifying agents, emulsifying agents, thickening agents, coating agents, gum bases, antifoaming agents, solvents, and improving agents. The additives may be selected according to food type, and they may be used in suitable amounts.

The health functional food of the present invention may be prepared by a method generally used in the art, and it may also be prepared by adding raw materials and ingredients which are generally added in the art during the preparation. Further, unlike other common drugs, the health functional food may be prepared using foods as raw materials, and thus it has the advantage of avoiding side effects associated with long-term administration of drugs, and it may be very portable.

Still another aspect of the present invention provides a feed composition for preventing or improving liver disease, including ginsenoside F2.

The ginsenoside F2 and liver disease are the same as described above.

The feed composition may include a feed additive. The feed additive of the present invention is classified as an auxiliary additive according to Control of Livestock and Fish Feed Act.

As used herein, the term "feed" means any natural or artificial diet, meal, etc., or components of such meals intended or suitable for being eaten, taken in, or digested by animals.

A kind of the feed may be, but is not particularly limited to, a feed generally used in the art. Non-limiting examples of the feed may include plant-based feeds, such as grains, nuts, food by-products, seaweeds, fibers, drug by-products, oil, starches, meals, and grain by-products; and animal-based feeds such as proteins, inorganic matters, fats, minerals, fats, single cell proteins, zooplanktons, and food, but are not limited thereto. These may be used alone or in a mixture of two or more thereof.

Hereinafter, the present invention will be described in detail with reference to the following Examples. However, these Examples are for illustrative purposes only, and the invention is not intended to be limited by these Examples.

### Example 1. Amelioration of alcohol-induced liver injury and Inhibition of fatty liver

50 mg/kg of ginsenoside F2 (indicated by GF2 in Drawing) dissolved in 3 g/kg of ethanol, together with a normal diet, were orally administered to 8-week-old male mice (weighing 25 g to 28 g) between 3 PM and 4 PM every day for 2 weeks. After 2 weeks, they were sacrificed, and the following experiment was performed.

A preparation method of ginsenoside F2 is as follows.

Leaves and roots of ginseng including Korean ginseng, American ginseng, and bamboo ginseng were extracted with twenty volumes of 80% ethanol twice or more, and dried to obtain crude saponins. The crude saponins were dissolved in water and adsorbed onto an HP-20 resin, and then washed with 100% water to remove carbohydrates. Thereafter, primary washing was performed using 40% ethanol to remove protopanaxatriol ginsenosides, Re and Rg1. Subsequently, washing was performed using 80% ethanol to elute protopanaxadiol ginsenosides, Rb1, Rb2, Rc, and Rd, which were then dried. The protopanaxadiol ginsenoside mixture was reacted as a substrate according to the method described in Korean Patent Publication No. 2013-0134930 to obtain at least 70% ginsenoside F2. Thereafter, ginsenoside F2 to be used as a feed was adsorbed onto an ODS resin, and then a proper concentration of ethanol was continuously applied in a concentration-gradient to obtain a ginsenoside F2 fraction with a purity of 95% or higher, which was dried and used.

### (1) Measurement of serum ALT, AST, TG, and total cholesterol levels

Blood was collected from mouse abdominal aorta and centrifuged using a centrifuge at 3,300 rpm for 10 minutes to separate only the serum. The levels of ALT (alanine aminotransferase) and AST (alanine aminotransferase) in the serum were measured using a kit of IDEXX Laboratories (USA) in accordance with the manufacturer's instructions by VetTEST (IDEXX, USA). Further, 50 mg of the hepatic tissue was excised and ground, and then a chloroform-methanol (2:1) solution was added thereto, followed by centrifugation. The lipid components contained in the hepatic tissue were extracted, and the levels of triglyceride (TG) and total cholesterol in the extract solution were measured using a kit of IDEXX Laboratories (USA) in accordance with the manufacturer's instructions by VetTEST.

Consequently, as shown in FIG. 2(a), administration of alcohol and vehicle (EtOH+Vehicle) increased the liver injury index ALT level by twice or more, whereas co-administration of alcohol and ginsenoside (EtOH+GF2) did not significantly increase the ALT level. As shown in FIG. 2(b), lower AST level was observed by administration of ginsenoside.

As shown in FIG. 2(c), a fatty liver index, TG level was also reduced to the normal level by co-administration of alcohol and ginsenoside (EtOH+GF2), showing the pattern similar to ALT.

### (2) Liver histological analysis

H&E (hematoxylin and eosin) staining was performed to observe histological changes in the liver tissue, and TUNEL (terminal deoxyribonucleotidyl transferase mediated dUTP nick end labeling) staining was performed to examine cell apoptosis. TUNEL staining was performed using an apoptosis detection kit (TAKARA BIO INC, Shiga, Japan) in accordance with the manufacturer's instructions. Further, triglycerides in hepatocytes were stained with Oil-red O. The results are shown in FIG. 3a at x200 or x400 magnification.

As a result, as shown in FIG. 3a, ethanol-induced inflammation was reduced by administration of ginsenoside F2 (H&E staining), and cell damage was also reduced by ginsenoside F2 (TUNEL staining). Triglyceride accumulation was also reduced by administration of ginsenoside F2 (Oil-red O staining).

FIG. 3b shows the results of statistical analysis of TUNEL-positive cells of FIG. 3a, in which TUNEL-positive cell death caused by alcohol administration was remarkably reduced by administration of ginsenoside F2. FIG. 3c shows the results of statistical analysis of triglycerides of FIG. 3a, in which triglycerides in the liver tissue were remarkably reduced by administration of ginsenoside F2.

### (3) Analysis of lipogenesis-related index in hepatocytes and hepatic stellate cells

Excessive consumption of alcohol causes an increase in 2-arachidonoylglycerol (2-AG), which is one of the endocannabinoids in the liver, and activates an endocannabinoid receptor (CB1R) which is a 2-arachidonoylglycerol receptor of hepatocytes, thereby increasing expression of a nuclear hormone receptor ERRγ. Further, the increased ERRγ directly binds to a transcription regulatory region of CYP2E1 to increase CYP2E1 expression, which in turn causes an increase in active oxygen, leading to liver injury (Kim DK et al., Gut. 2013 Jul;62(7):1044-54).

As shown in FIG. 4a, when hepatocytes separated from mouse liver were administered with alcohol and vehicle (EtOH+Vehicle), expression of the liver injury-related factors CYP2e1, ADH1, CB1R, SREBP1c, and FAS was increased, but expression of these factors was significantly reduced by administration of ginsenoside (EtOH+GF2). In particular, expression of SREBP1c and FAS, which are transcription factors regulating a fat accumulation pathway in the liver, was reduced by administration of ginsenoside F2, and expression of CB1R, which is known to be critical for fat synthesis in hepatocytes, was also reduced.

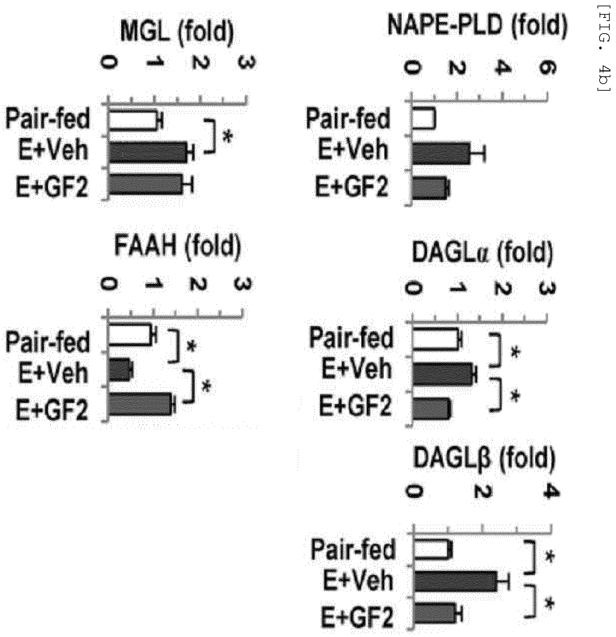

As shown in FIG. 4b, when hepatic stellate cells separated from mouse liver were administered with alcohol and vehicle (EtOH+Vehicle), expression of the liver injury-related factors NAPE-PLD, DAGLα,β, and MGL was increased, and expression of FAAH was reduced, whereas expression of DAGL-α,β, which is involved in endocannabinoid synthesis to induce fat synthesis, was significantly reduced and fatty acid amide hydrolase (FAAH) was significantly increased by administration of ginsenoside (EtOH+GF2).

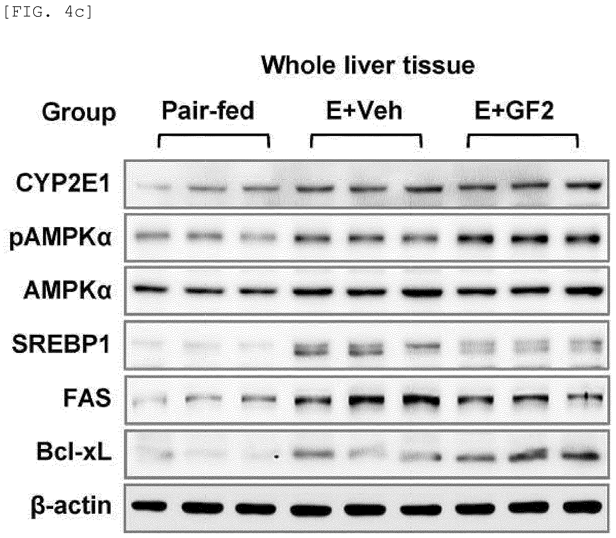

FIG. 4c shows the results of Western blotting of proteins extracted from the liver tissues. Expression of fat oxidation-inducing pAMPK was increased and expression of lipogenic SREBP1c and FAS was reduced by administration of ginsenoside F2.

Taken together, ginsenoside F2 of the present invention was found to have the effect of regulating the fat metabolism in the liver to inhibit fatty liver formation and to ameliorate liver injury.

### Example 2. Test in co-cultured hepatocytes and hepatic stellate cells

### (1) Inhibition of fat synthesis in hepatocytes and hepatic stellate cells

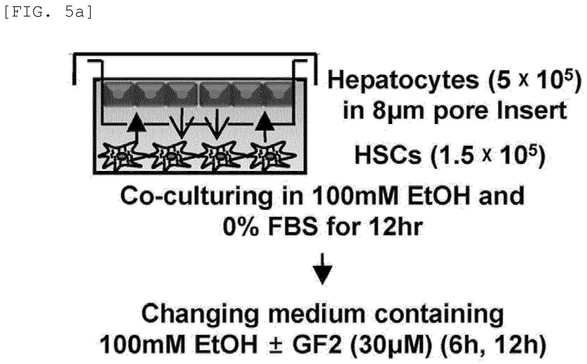

Hepatocytes and hepatic stellate cells were isolated from mice, and then, as shown in FIG. 5a, hepatic stellate cells (HSC) were placed in the bottom and hepatocytes were placed thereon to perform co-culture. During the culture, they were treated with 100 mM ethanol for 12 hours, and then 30 µM ginsenoside F2 for 6 hours or 12 hours.

FIG. 5b shows the results of Western blotting of the co-cultured hepatocytes. Unlike hepatocytes treated with only ethanol, hepatocytes treated with ethanol and ginsenoside F2 showed increased expression of lipolytic pAMPK and decreased expression of lipogenic SREBP1c and FAS.

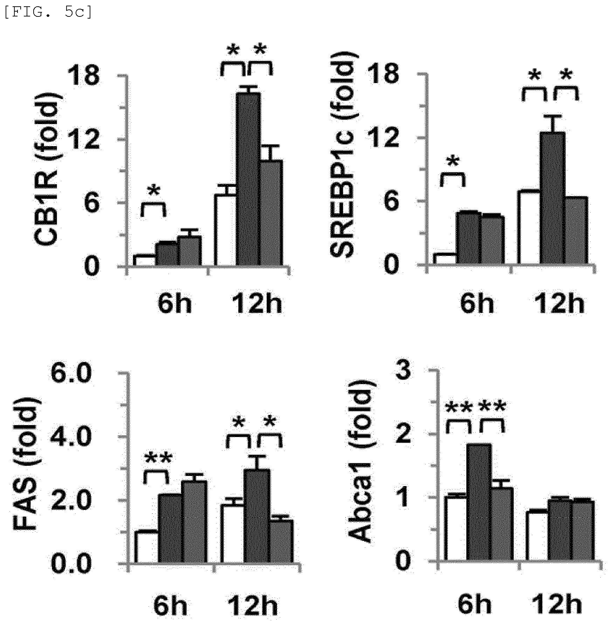

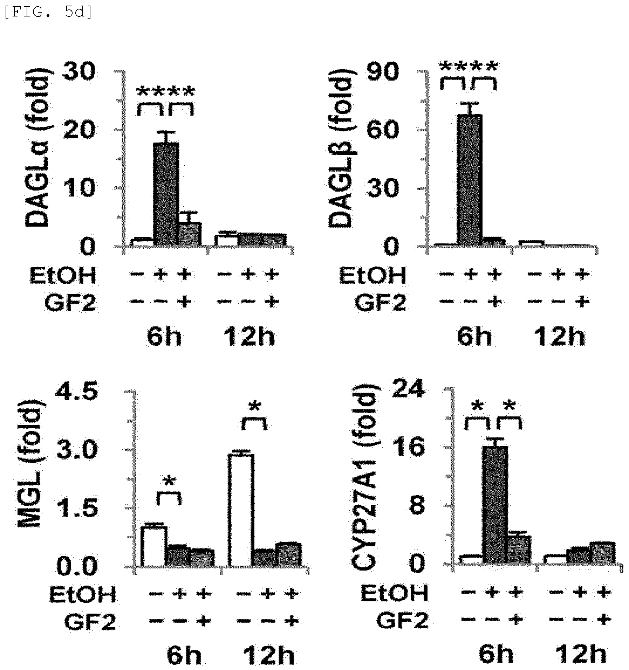

FIG. 5c shows the results of real-time PCR of the co-cultured hepatocytes. Treatment of ginsenoside F2 reduced expression of CB1R, SREBP1c and FAS, which play an important role in fat synthesis. When the co-cultured hepatic stellate cells were treated with ginsenoside F2, expression of DAGL-α,β, which is involved in endocannabinoid synthesis to induce fat synthesis, was reduced (FIG. 5d).

### (2) Inhibition of LXR-mediated fat synthesis and endocannabinoid production

To examine a fat synthesis inhibition mechanism of ginsenoside F2, human hepatocyte cell line HepG2A was treated with 30 µM ginsenoside F2, and 30 minutes later, further treated with 10 µM TO901317, which induces fat synthesis as an LXR agonist stimulating fatty acid synthesis, followed by culture for 9 hours. Thereafter, real-time PCR of hepatocytes was performed, and as a result, ginsenoside F2 was found to inhibit expression of lipogenic genes, SREBP1c and FAS increased by TO901317 (FIG. 5e).

Further, HepG2A was treated with ginsenoside F2 by varying its concentration from 0 µM to 100 µM, and cultured for 12 hours. As a result, expression of SREBP1c and FAS was reduced, and in particular, an excellent inhibitory effect was observed at the concentration of 40 µM or higher (FIG. 5f).

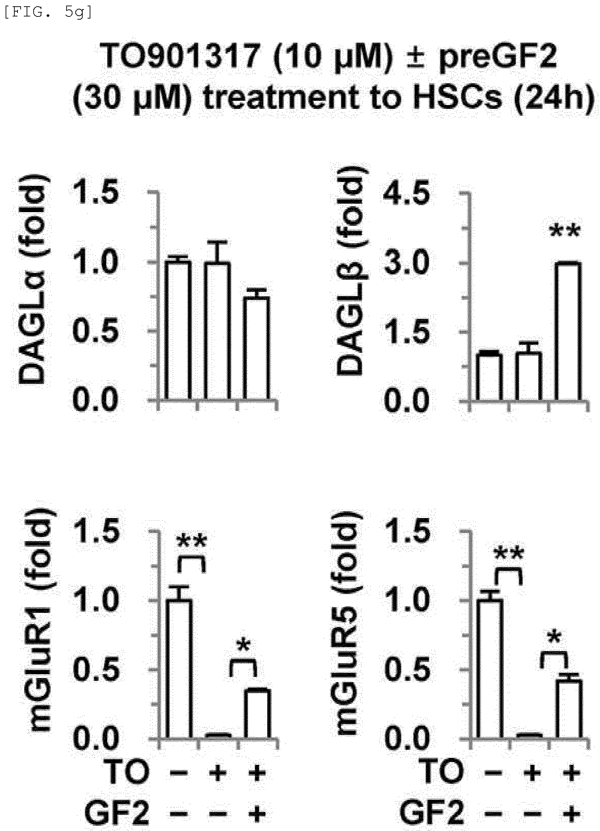

Hepatic stellate cells were treated with 30 µM of ginsenoside F2, and 30 minutes later, further treated with 10 µM TO901317, followed by culture for 12 hours. FIGS. 5f and 5g show the results of real-time PCR and Western blotting of the cultured hepatic stellate cells. As a result, when the cells were treated with fat synthesis-stimulating TO901317, a glutamate receptor mGluR1 or 5 was hardly expressed, whereas its expression was significantly increased by treatment of ginsenoside F2.

Taken together, it was confirmed that ginsenoside F2 of the present invention exhibits excellent inhibitory effects on fat synthesis in hepatocytes and hepatic stellate cells, suggesting that the inhibition of fat synthesis in the liver is attributed to a mechanism in which ginsenoside F2 inhibits alcohol-mediated endocannabinoid generation to reduce CB1R signal transduction in adjacent hepatocytes.

### Example 3. Inhibition of hepatic inflammatory changes by increased regulatory T cells

Mice were fed with 3 g/kg of ethanol together with a normal diet every day for 2 weeks. In this regard, Group 1 (E+Veh) was fed with vehicle, and Group 2 (E+GF2) was fed with ginsenoside F2.

Thereafter, monocytes were isolated from the liver, and stained with the macrophage markers, CD11b and F4/80, followed by flow cytometry. As a result, treatment of ginsenoside F2 reduced a percentage of macrophage (FIG. 6a). Flow cytometry was also performed using the neutrophil markers CD11b and Gr-1. As a result, treatment of ginsenoside F2 reduced a percentage of neutrophil (FIG. 6b). Macrophage and neutrophil are known to be closely related to inflammation, and therefore, these results suggest that ginsenoside F2 has a possibility of ameliorating inflammation in hepatocytes.

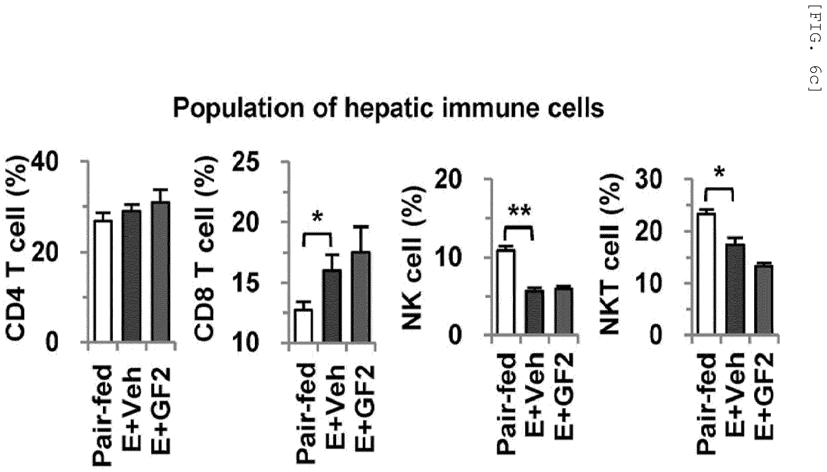

Further, inflammatory cells in the liver were stained with regulatory T cell (Treg) markers, CD4, CD25, and Foxp3. As a result, treatment of ginsenoside F2 increased regulatory T cells, and decreased Th17 cells (IL-17 and CD4 positive) which are one of proinflammatory cells. As shown in FIG. 6c, however, ginsenoside F2 exhibited no significant effects on the percentages of CD4, CD8 T cells, NK cells, and NKT cells.

Further, the isolated monocytes were subjected to real-time PCR. As a result, expression of the inflammatory cytokines TNF-α, IL-6, and IL-17 increased by alcohol was inhibited by ginsenoside F2, and expression of anti-inflammatory cytokine IL-10 was increased thereby (FIG. 6d). These effects are consistent with the results of ELISA of mouse serum (FIG. 6e).

Taken together, it was found that ginsenoside F2 of the present invention increases distribution of regulatory T cells which inhibits activity of inflammatory cells such as macrophage or neutrophil in alcohol-induced hepatitis, and also increases expression of anti-inflammatory cytokine IL-10 in regulatory T cells, thereby ameliorating hepatic inflammation. Therefore, ginsenoside F2 may be used for the prevention or treatment of liver disease.

### Example 4. Inhibition of differentiation of naive T cells into IL-17-producing Th17 cells

Naive T cells were isolated from mice, and treated with 5 ng/mL TGF-β1 and 20 ng/mL IL-6, and then cultured in a CD3/28 antibody-coated dish for 3.5 days to differentiate the cells into proinflammatory Th17 cells (T helper 17 cells). In this regard, the cells were also treated with ginsenoside F2 by varying its concentration (0 µM to 30 µM), and markers for Th17 cells were examined, and the results are shown in FIGS. 7a to 7c. N represents naive T cells treated with none of TGF-β1, IL-6, and ginsenoside F2.

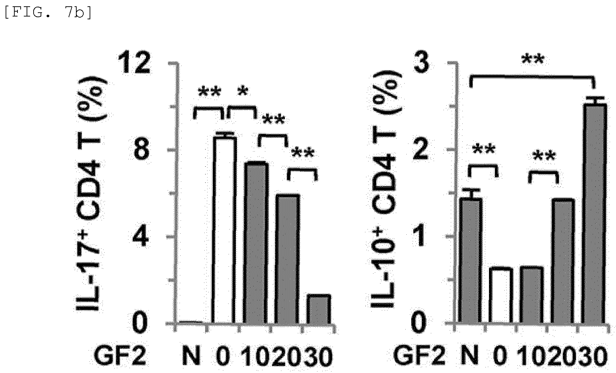

FIGS. 7a and 7b show the results of FACS of differentiated naive T cells, in which IL-17-expressing Th17 cells were decreased in a ginsenoside F2 concentration dependent manner, whereas anti-inflammatory cytokine IL-10 was increased in a ginsenoside F2 concentration dependent manner.

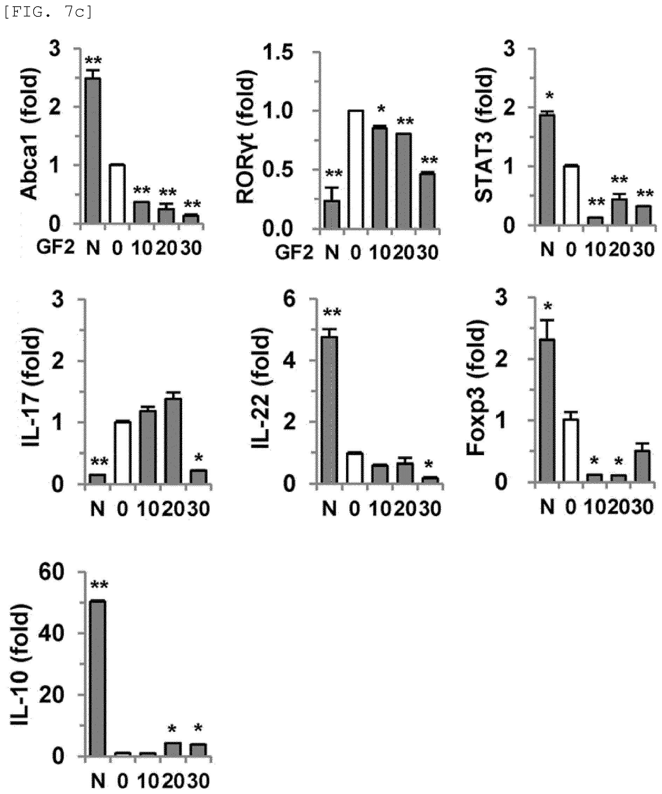

Further, real-time PCR of the differentiated cells was performed. As shown in FIG. 7c, expression of Abcal, which selectively lowers HDL cholesterol without increasing triglyceride, was decreased in a ginsenoside F2 concentration dependent manner, and Th17 cell-specific transcription factors RORγt (ROR gamma t) and STAT3 were also decreased by treatment of ginsenoside F2.

Taken together, it was found that ginsenoside F2 of the present invention inhibits differentiation of naive T cells into proinflammatory Th17 cells to ameliorate hepatic inflammation caused by factors other than alcohol, and are thereby used for the treatment of liver disease.

### Example 5. Loss of liver-protective effect of GF2 in IL-10-deficient mice

8-week-old IL-10-deficient male mice (weighing 25 g to 28 g) were orally administered with 3 g/kg ethanol and 50 mg/kg ginsenoside F2, together with a normal diet, every day for 2 weeks (E+GF2). A control group was fed with an equal amount of ethanol and vehicle instead of ginsenoside F2 (E+Veh). 2 weeks later, the sera, liver tissues, and hepatic monocytes were obtained from the mice by autopsy. Thereafter, in the same manner as in Example 1. (1), the serum ALT, AST, TG, and cholesterol levels were measured, and necrotic foci were examined in the liver tissue to count the number of necrotic foci in two sections of the liver. In the hepatic monocytes, macrophage and neutrophil expression levels were examined by flow cytometry using F4/80 or Gr1 antigen marker.

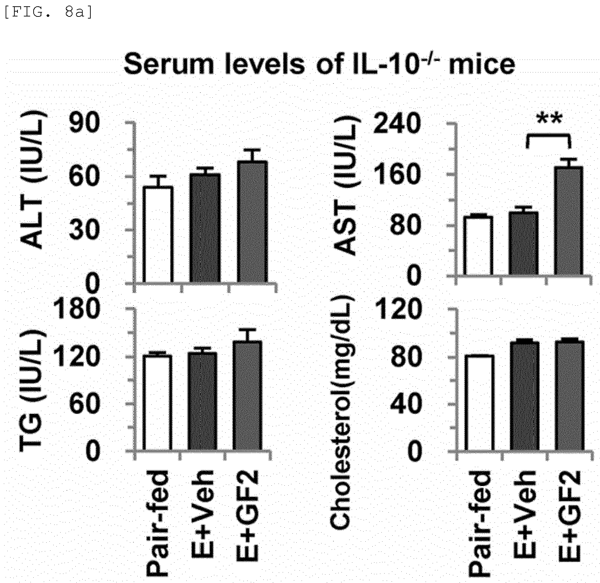

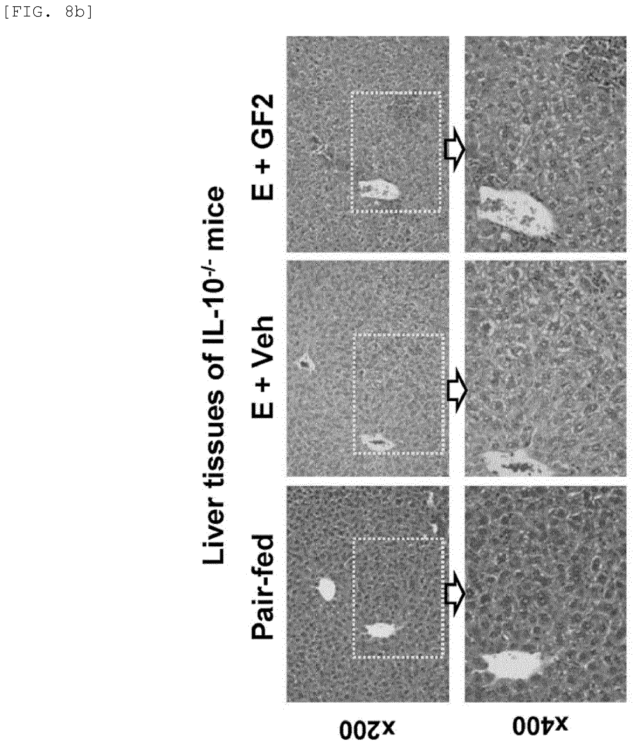

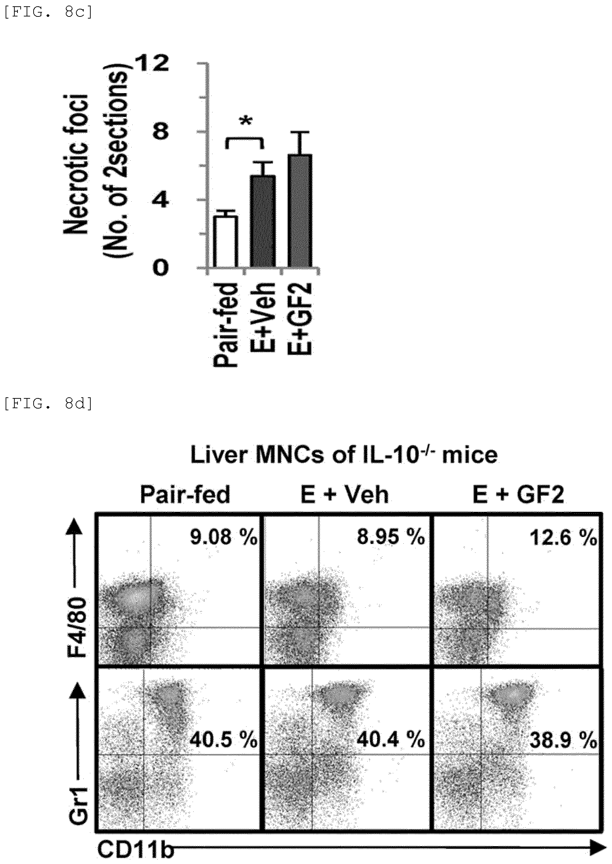

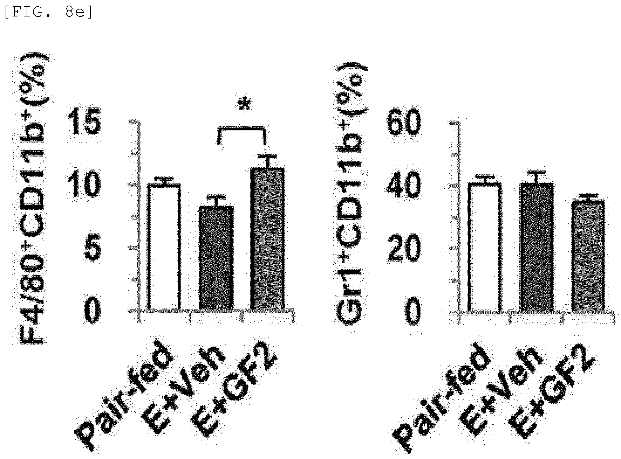

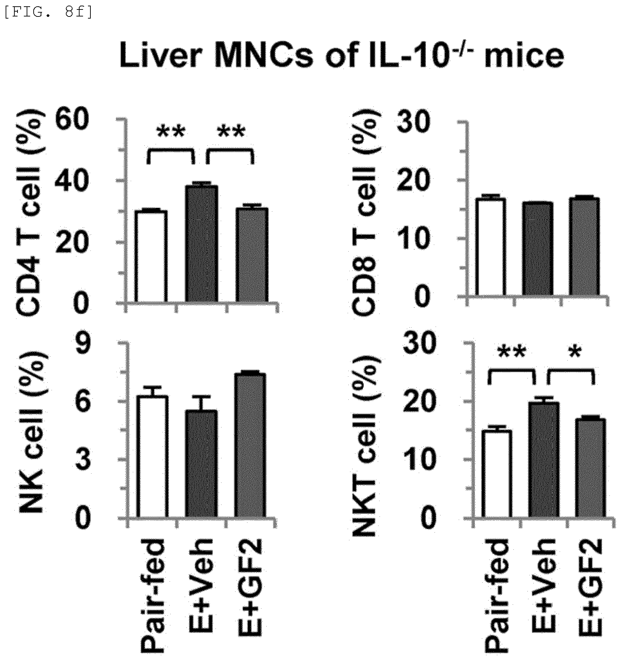

As a result, even though IL-10-deficient mice were administered with ginsenoside F2, no significant effects were observed in ALT, TG, and cholesterol levels (FIG. 8a), and no great changes were found in the necrotic foci of the liver tissue (FIGS. 8b and 8c). Further, ginsenoside F2 exhibited no significant effects on expression of macrophage and neutrophil (FIGS. 8d and 8e) and other immune cells (FIG. 8f).

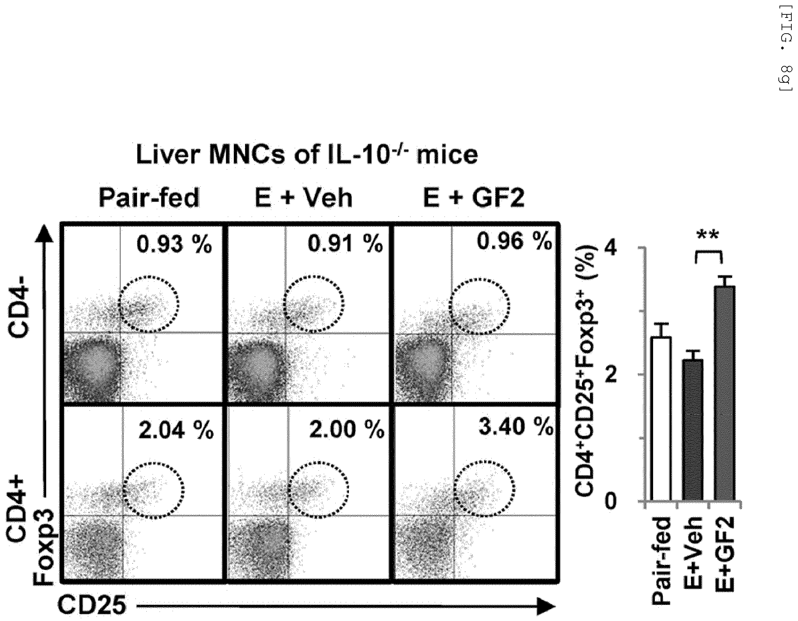

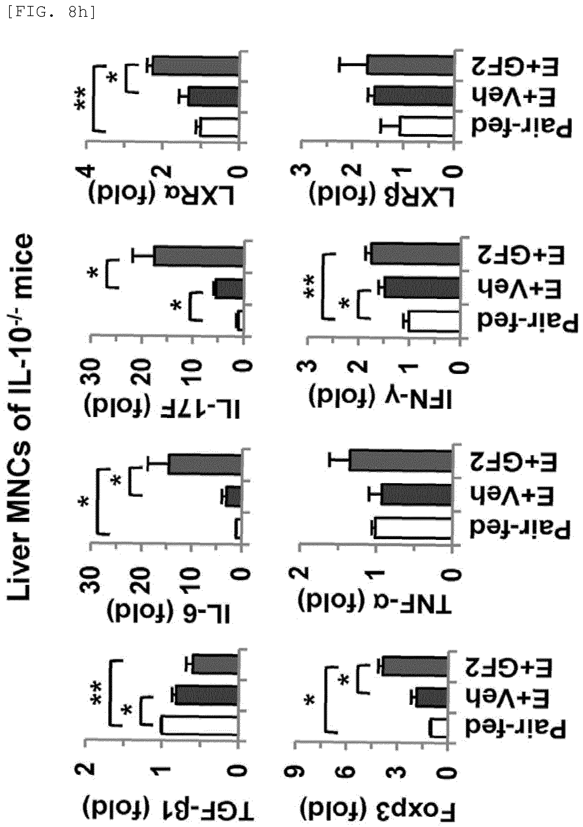

However, CD4⁺CD25⁺Foxp3⁺ regulatory T cells were increased in the ginsenoside F2-treated group (FIGS. 8g), and the results of real-time PCR using monocytes showed that expression of inflammatory cytokines IL-6 and IL-17 and expression of Foxp3 were increased in the ginsenoside F2-treated group, as shown in FIG. 8h.

Taken together, the liver protective effect of ginsenoside F2 was hardly observed in IL-10-deficient mice, implying that the liver protective effect occurs during production of regulatory T cells (Tregs) and anti-inflammatory cytokine IL-10 by ginsenoside F2.

In Examples, data shown are presented as mean±SEM; *P < 0.05, **P < 0.01 versus the corresponding control.

Based on the above description, it will be understood by those skilled in the art that the present invention may be implemented in a different specific form without changing the technical spirit or essential characteristics thereof. Therefore, it should be understood that the above embodiment is not limitative, but illustrative in all aspects. The scope of the invention is defined by the appended claims rather than by the description preceding them, and therefore all changes and modifications that fall within metes and bounds of the claims, or equivalents of such metes and bounds, are therefore intended to be embraced by the claims.

### Effect of the invention

The pharmaceutical composition including ginsenoside F2 according to the present invention inhibits fat synthesis and accumulation in the liver, increases distribution of regulatory T cells capable of inhibiting activity of inflammatory cells, increases expression of anti-inflammatory cytokine IL-10 in regulatory T cells, and inhibits differentiation of naive T cells into proinflammatory Th17 cells, and is thereby effectively used for the prevention or treatment of liver disease.

## Claims

1. A pharmaceutical composition comprising ginsenoside F2, for use in the prevention or treatment of alcoholic liver disease.

2. The pharmaceutical composition for use of claim 1, wherein the liver disease is selected from the group consisting of hepatitis, cirrhosis, fatty liver, hepatic insufficiency, and liver cancer.

3. The pharmaceutical composition for use of claim 1, wherein ginsenoside F2 inhibits fat accumulation in the liver.

4. The pharmaceutical composition for use of claim 1, wherein ginsenoside F2 increases distribution of regulatory T cells (Tregs) to inhibit hepatic inflammation.

5. The pharmaceutical composition for use of claim 1, wherein ginsenoside F2 increases expression of anti-inflammatory cytokine IL-10.

6. The pharmaceutical composition for use of claim 1, wherein ginsenoside F2 inhibits differentiation of naive T cell into Th17 cells.

7. The pharmaceutical composition for use of claim 1, wherein ginsenoside F2 inhibits activity of LXR alpha (liver X receptor alpha).

## Patentansprüche

1. Pharmazeutische Zusammensetzung umfassend Ginsenosid F2, zur Verwendung bei der Verhinderung oder Behandlung von alkoholischer Lebererkrankung.

2. Pharmazeutische Zusammensetzung zur Verwendung gemäß Anspruch 1, wobei die Lebererkrankung ausgewählt ist aus der Gruppe bestehend aus Hepatitis, Zirrhose, Fettleber, Leberinsuffizienz, und Leberkrebs.

3. Pharmazeutische Zusammensetzung zur Verwendung gemäß Anspruch 1, wobei Ginsenosid F2 Fettakkumulation in der Leber inhibiert.

4. Pharmazeutische Zusammensetzung zur Verwendung gemäß Anspruch 1, wobei Ginsenosid F2 die Verteilung von regulatorischen T Zellen (Tregs) steigert um Leberentzündung zu inhibieren.

5. Pharmazeutische Zusammensetzung zur Verwendung gemäß Anspruch 1, wobei Ginsenosid F2 die Expression von anti-entzündlichem Zytokin IL-10 steigert.

6. Pharmazeutische Zusammensetzung zur Verwendung gemäß Anspruch 1, wobei Ginsenosid F2 die Differenzierung naiver T Zelle in Th17 Zellen inhibiert.

7. Pharmazeutische Zusammensetzung zur Verwendung gemäß Anspruch 1, wobei Ginsenosid F2 die Aktivität von LXR alpha (Leber X Rezeptor alpha (*liver X receptor alpha*)) inhibiert.

## Revendications

1. Composition pharmaceutique comprenant du ginsénoside F2, pour une utilisation dans la prévention ou le traitement d'une maladie hépatique alcoolique.

2. Composition pharmaceutique pour une utilisation selon la revendication 1, dans laquelle la maladie du foie est choisie dans le groupe constitué par l'hépatite, la cirrhose, la stéatose hépatique, l'insuffisance hépatique et le cancer du foie.

3. Composition pharmaceutique pour une utilisation selon la revendication 1, dans laquelle le ginsénoside F2 inhibe l'accumulation de graisse dans le foie.

4. Composition pharmaceutique pour une utilisation selon la revendication 1, dans laquelle le ginsénoside F2 augmente la distribution des lymphocytes T régulateurs (Trég) pour inhiber l'inflammation hépatique.

5. Composition pharmaceutique pour une utilisation selon la revendication 1, dans laquelle le ginsénoside F2 augmente l'expression de la cytokine anti-inflammatoire IL-10.

6. Composition pharmaceutique pour une utilisation selon la revendication 1, dans laquelle le ginsénoside F2 inhibe la différenciation des lymphocytes T naïfs en cellules Th17.

7. Composition pharmaceutique pour une utilisation selon la revendication 1, dans laquelle le ginsénoside F2 inhibe l'activité du LXR alpha (récepteur X du foie alpha).
